# FASCICULE DE BREVET EUROPEEN

(11) **EP 1 605 995 B1**
(45) Date de publication et mention de la délivrance du brevet: **31.10.2018**
(21) Numéro de dépôt: 04742277.9
(22) Date de dépôt: 18.03.2004
(51) Int. Cl.: A61M 5/46, A61M 5/48, A61M 5/30, A61M 5/20

(54) **DISPOSITIF D´INJECTION SANS AIGUILLE A MOYENS DE REGULATION DU NIVEAU DE LA PRESSION DES GAZ DANS LA CHAMBRE DE COMBUSTION**
NADELLOSE INJEKTIONSVORRICHTUNG MIT EINEM GASDRUCKREGELSYSTEM IN DER BRENNKAMMER
NEEDLELESS INJECTION DEVICE COMPRISING MEANS FOR REGULATING THE GAS PRESSURE LEVEL IN THE COMBUSTION CHAMBER

(30) Priorité: 21.03.2003 FR 0303496
(43) Date de publication de la demande: 21.12.2005
(73) Titulaire: Crossject, 21000 Dijon (FR)
(72) Inventeur: ALEXANDRE, Patrick, F-70100 Gray (FR); BROUQUIERES, Bernard, F-83100 Toulon (FR); DESAILLY, David, F-91710 Vert Le Petit (FR)
(74) Mandataire: Gaillarde, Frédéric F. Ch.
(86) Numéro de dépôt international: PCT/FR2004/000659
(87) Numéro de publication internationale: WO 2004/084977

(56) Documents cités:
- WO-A-00/44421
- WO-A-01/97880
- US-B1- 6 258 063

## Description

Le domaine technique de l'invention est celui des dispositifs d'injection sans aiguille préremplis et jetables, fonctionnant avec un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Lorsqu'un dispositif d'injection sans aiguille utilise, pour l'injection d'un principe actif liquide, un générateur pyrotechnique de gaz, les contraintes mécaniques, thermiques et dynamiques engendrées sur le dispositif par les gaz issus de la combustion de la charge pyrotechnique du générateur sont très importantes. La présence de ces contraintes impose donc de disposer d'un dispositif suffisamment résistant pour fonctionner de manière fiable et notamment pour ne pas perturber l'injection du principe actif à travers la peau du patient.

Le dispositif d'injection sans aiguille peut être rendu résistant et sans danger pour son utilisateur en étant fabriqué à partir de matériaux eux-mêmes résistants. Cependant ces matériaux peuvent s'avérer coûteux et lourds. Leur utilisation augmentera donc nécessairement le coût de fabrication du dispositif et la masse de ce dispositif. Or, il est évident que le coût de fabrication d'un dispositif d'injection sans aiguille jetable doit rester le plus faible possible et qu'un tel dispositif doit rester maniable pour être facilement utilisable par la plupart des individus et notamment les personnes âgées.

Il convient donc de proposer un dispositif d'injection sans aiguille jetable qui soit léger, maniable, d'un coût de fabrication faible et dans lequel, également, les contraintes mécaniques, thermiques et dynamiques sont limitées.

Le document WO 00/44421 décrit un dispositif d'injection sans aiguille correspondant au préambule de la revendication 1 ci-annexée.

D'autres documents, notamment US 6 258 063 et WO 01/97880 décrivent des seringues sans aiguille faisant partie de l'arrière-plan technologique.

La présente invention vise à améliorer le dispositif selon WO 00/44421, et notamment à limiter le risque que les gaz de pollution ne viennent polluer le principe actif.

On atteint ce but avec les caractéristiques de la partie caractérisante de la revendication 1 ci-annexée : la présence d'une membrane expansible délimitant le troisième volume de la chambre de combustion permet de limiter les risques de pollution du principe actif contenu dans le réservoir par les gaz de combustion.

Selon une autre particularité, le premier dispositif de régulation du niveau de la pression est constitué d'un passage formé à travers la paroi. Ainsi le débit des gaz arrivant dans le réservoir pour pousser le principe actif est contrôlé et maîtrisé ce qui permet de limiter les contraintes thermiques, dynamiques et mécaniques sur le réservoir lors du fonctionnement du dispositif et d'éviter ainsi d'endommager le dispositif et de blesser son utilisateur.

Selon une autre particularité, la charge pyrotechnique est disposée dans un premier sous-volume du premier volume de la chambre de combustion, ce premier sous-volume étant initialement fermé.

Selon une autre particularité, le premier sous-volume du premier volume de la chambre de combustion est séparé, par un deuxième dispositif de régulation du niveau de la pression, d'un deuxième sous-volume du premier volume de la chambre de combustion, situé en aval par rapport au premier sous-volume. Par exemple, si la combustion de la charge pyrotechnique est incomplète ou mauvaise, le principe actif ne pénétrera pas correctement à travers la peau et à la profondeur nécessaire. Selon l'invention, en utilisant ce deuxième dispositif de régulation, il s'agit donc de s'assurer de la parfaite combustion de la charge pyrotechnique et de maintenir la charge pyrotechnique dans un volume fermé jusqu'à ce que la quasi-totalité et même idéalement que la totalité de la charge pyrotechnique soit brûlée.

Selon une autre particularité, le deuxième dispositif de régulation est constitué d'un opercule frangible calibré. L'opercule s'ouvrira par exemple en pétales suivant une amorce de rupture, les pétales restant solidaires de l'opercule après ouverture. De cette manière, on évite la projection de particules chaudes et agressives dans la chambre de combustion.

Selon une autre particularité, le premier sous-volume du premier volume, dans lequel est placée la charge pyrotechnique, est délimité en partie par les parois d'une cartouche insérée dans le corps du dispositif. Ce premier sous-volume est donc formé indépendamment du corps du dispositif. Selon l'invention, en utilisant une cartouche génératrice de gaz indépendante, il est possible d'adapter, lors du processus d'assemblage du dispositif, la quantité de charge pyrotechnique à la nature et/ou à la quantité de principe actif à injecter ainsi qu'à la profondeur de pénétration souhaitée pour ledit principe actif.

Selon une autre particularité, la charge pyrotechnique est placée dans la cartouche entre l'opercule frangible calibré et une amorce apte à initier la charge pyrotechnique.

Selon une autre particularité, la cartouche a la forme d'un conduit en L dans lequel est placée la charge pyrotechnique, ce conduit étant obturé à l'une de ses extrémités par l'amorce et à son autre extrémité par l'opercule frangible calibré. Cette forme est particulièrement adaptée pour un dispositif d'injection sans aiguille de forme compacte.

Selon une autre particularité, la membrane se déploie dans le réservoir de principe actif.

Selon l'invention, étant donné la nécessité de conserver un principe actif propre lors du fonctionnement du dispositif, cette membrane est interposée entre la chambre de combustion et le réservoir pour éviter que les gaz de combustion ne viennent polluer le principe actif. La membrane devra être constituée d'un matériau suffisamment souple et suffisamment résistant pour pouvoir se déployer sous l'action des gaz et venir pousser le principe actif compris dans le réservoir.

Selon une autre particularité, le passage est décalé par rapport à un axe central longitudinal de la chambre de combustion et est formé de sorte à être le plus éloigné possible de la membrane. Pour éviter que les gaz chauds et agressifs sortant du premier volume viennent directement au contact de la membrane initialement repliée et l'endommagent, le passage e déporté par rapport à l'axe de la chambre de combustion.

Selon une autre particularité, le circuit d'éléments suit une forme en U renversé comprenant donc deux branches parallèles reliées entre elles par une branche transversale. Ce type d'architecture confère au dispositif une forme compacte, ergonomique et peu encombrante.

L'invention, avec ses caractéristiques et avantages, ressortira plus clairement à la lecture de la description faite en référence aux dessins annexés dans lesquels :
La figure 1 représente en perspective et en mode éclaté le corps du dispositif ainsi que certains
   éléments destinés à être assemblés sur le corps du dispositif.
La figure 2 représente en perspective le corps du dispositif sur lequel certains éléments ont été assemblés ainsi que la cartouche génératrice de gaz.
La figure 3 représente en perspective et en mode éclaté le réservoir destiné à recevoir le principe actif liquide.
La figure 4 représente en perspective et en mode éclaté une cartouche pyrotechnique génératrice de gaz utilisée dans le dispositif selon l'invention.
La figure 5 représente en perspective un opercule frangible tel qu'utilisé dans la cartouche pyrotechnique de la figure 4.
Les figures 6A et 6B représentent en coupe longitudinale le corps du dispositif respectivement avant fonctionnement et après fonctionnement. Sur ces figures 6A et 6B sont représentés plus particulièrement les différents volumes de la chambre de combustion du dispositif.
La figure 7 représente en coupe longitudinale partielle un dispositif d'injection sans aiguille selon l'invention, non actionné, dans lequel est insérée la cartouche pyrotechnique représentée en figure 4.

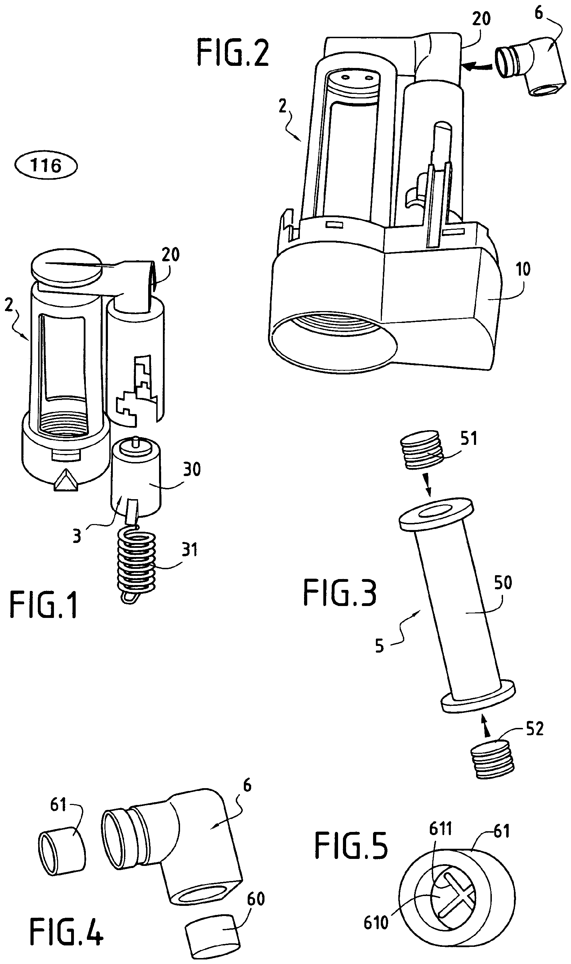

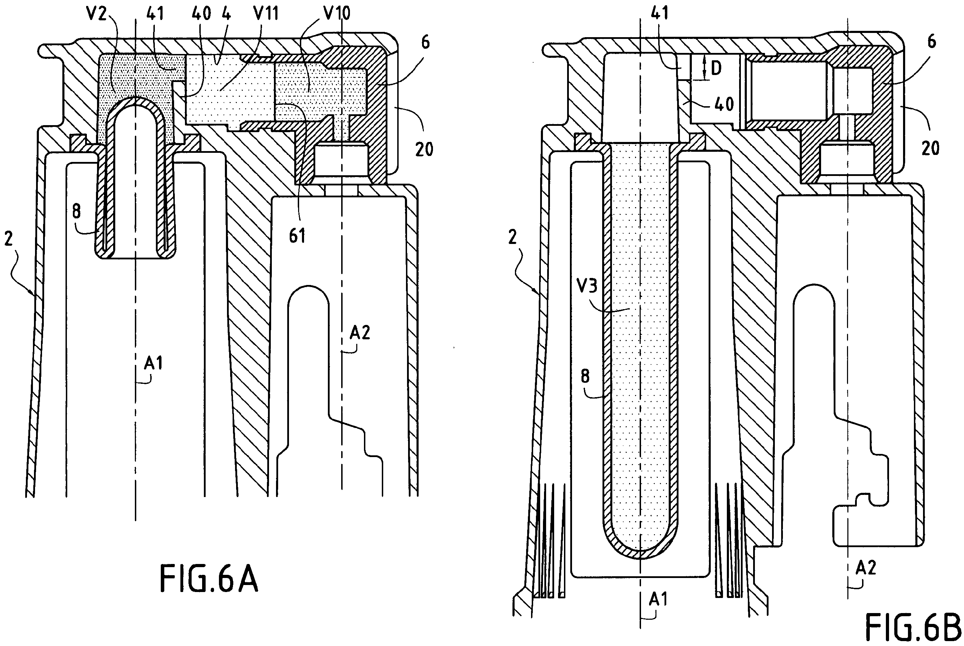

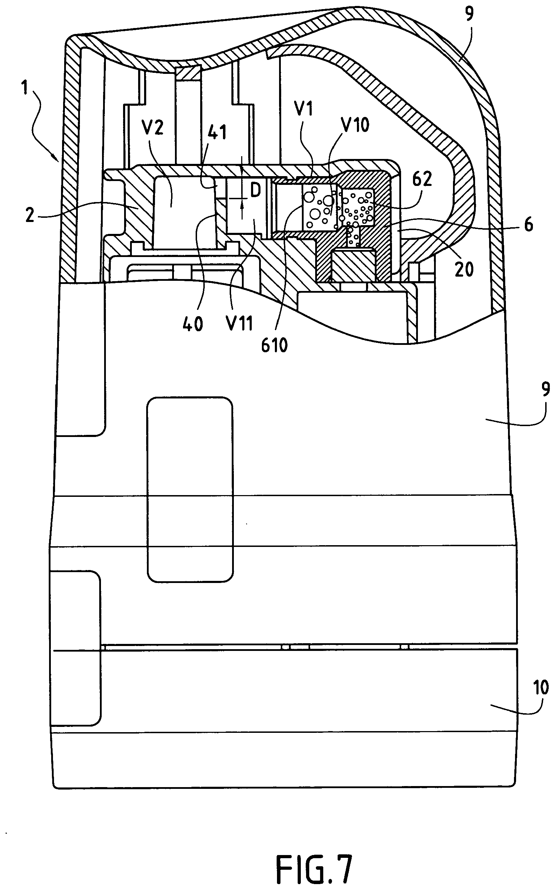

Un dispositif 1 d'injection sans aiguille selon l'invention, représenté en figure 7, comporte un corps 2 creux en forme de U renversé inséré sous un capot 9 d'actionnement du dispositif 1, ce capot étant obturé par un bouchon 10. Cette forme en U confère au dispositif une forme compacte dont les avantages sont plus particulièrement décrits dans le brevet n° FR 2 815 544. L'actionnement d'un tel dispositif 1 par le patient, à l'aide du capot 9, est également décrit dans le brevet FR 2 815 544. Lors du processus d'assemblage du dispositif 1, ce corps 2 est destiné à recevoir une pluralité d'éléments. Ainsi, une fois assemblé, le corps 2, représenté en figure 1, comporte ou délimite successivement, de l'amont vers l'aval, un dispositif 3 de percussion comprenant un percuteur 30 et un ressort 31, une amorce 60, une charge 62 (Figure 7) pyrotechnique, ces trois éléments formant un générateur de gaz, une chambre 4 de combustion, un réservoir 5 (figure 3) contenant un principe actif liquide à injecter et un système d'injection (non visible). Le générateur de gaz constitue un premier sous-ensemble linéaire inséré dans le corps 2 suivant une première branche verticale du U renversé formé par le corps 2. Le réservoir 5 contenant le principe actif à injecter et le système d'injection forment un second sous-ensemble linéaire inséré suivant la seconde branche verticale du U renversé formé par le corps 2. Le premier et le second sous-ensembles sont linéaires suivant deux axes (A1, A2, figures 6A et 6B) parallèles et sont reliés entre eux par la chambre 4 de combustion qui est formée dans le corps 2 suivant un axe perpendiculaire aux axes (A1, A2) des deux sous-ensembles, c'est-à-dire suivant la branche transversale reliant les deux branches parallèles du U renversé formé par le corps 2.

Le réservoir 5 représenté en figure 3 est par exemple constitué d'un tube 50 en verre ouvert à ses deux extrémités. Le tube 50 est inséré dans le corps 2 de manière à être relié, à son extrémité la plus en amont, à la chambre 4 de combustion et à son extrémité la plus en aval au système d'injection. Le principe actif (non représenté) est par exemple emprisonné dans le tube 50 en verre entre un bouchon-piston amont 51 et un bouchon-piston aval 52 enfoncés dans le tube 50. Les bouchons-pistons amont 51 et aval 52 sont réalisés par exemple dans un matériau déformable à base d'élastomère. Le système d'injection comporte notamment une buse d'injection à travers laquelle est injecté le principe actif contenu dans le réservoir 5. Cette buse d'injection comprend par exemple une pluralité de canaux d'injection destinés à être traversés par le liquide lors de l'injection.

Selon l'invention, la chambre 4 de combustion est divisée en une pluralité de volumes V1,V2 adjacents successifs ayant chacun une fonction bien déterminée. Ces volumes sont formés suivant l'axe défini par la branche transversale du U renversé formé par le corps 2.

Une paroi 40 transversale située dans la chambre 4 de combustion divise la chambre 4 de combustion en deux volumes V1,V2 distincts formés dans le corps. Un ajutage 41 ou passage, d'un diamètre (D, figures 6B et 7) déterminé, est formé sur la paroi 40 pour faire communiquer les deux volumes V1,V2. Le premier volume V1 défini comme le volume situé le plus en amont est divisé lui-même en deux sous-volumes V10,V11. Dans un premier sous-volume V10 situé le plus en amont est placée la charge 62 pyrotechnique génératrice de gaz. Plus précisément, ce premier sous-volume V10 est défini dans une cartouche 6 génératrice de gaz dans laquelle est placée la charge 62 pyrotechnique. La cartouche 6 est insérée dans un logement spécifique prévu pour elle dans la chambre 4 de combustion. Ce logement suit l'angle droit défini entre la chambre 4 de combustion des gaz et la première branche verticale du U formé par le corps 2. Une ouverture 20 communiquant avec le logement est formée sur le corps 2. Cette ouverture 20 est formée latéralement sur le corps 2, sensiblement dans l'axe de la chambre 4 de combustion des gaz.

Selon l'invention, le générateur de gaz comporte donc une cartouche 6 génératrice de gaz insérée dans le corps 2 du dispositif 1 par une ouverture 20 spécifique formée sur le corps 2. La cartouche 6 est ensuite sertie sur le corps 2 au niveau de l'ouverture 20. La cartouche 6 génératrice de gaz représentée en figures 2 et 4 est par exemple métallique et comporte une amorce 60 et une charge 62 (Figure 7) pyrotechnique permettant de générer la quantité de gaz nécessaire pour provoquer l'injection du principe actif. L'amorce 60 est par exemple du type de celle utilisée dans une cartouche pour fusil de chasse. La charge 62 pyrotechnique est constituée d'une poudre apte à émettre une grande quantité de gaz comme, par exemple, une poudre simple base à la nitrocellulose. En référence à la figure 4, la cartouche 6 génératrice de gaz utilisée dans le dispositif 1 d'injection sans aiguille selon l'invention se présente par exemple sous la forme d'un conduit en forme de L dans lequel est placée la charge 62 pyrotechnique. Lorsque la cartouche 6 est encastrée dans le dispositif 1 comme représenté en figure 7, sa forme en L suit l'angle droit formé entre la première branche verticale du U renversé formé par le corps 2 et sa branche transversale. En outre, une fois la cartouche 6 en place dans le logement, l'extrémité la plus en amont du conduit formant la cartouche 6 est obturée par l'amorce 60 tandis que l'extrémité la plus en aval du conduit est obturée par un opercule 61 frangible calibré. L'opercule 61 frangible obturant le conduit en L formé par la cartouche 6 à son extrémité aval se trouve alors dans l'axe de la chambre 4 de combustion et l'amorce 60 obturant ledit conduit à son extrémité amont se trouve dans l'axe du premier sous-ensemble et plus particulièrement dans l'axe du percuteur 30.

L'opercule 61 frangible, représenté plus en détail en figure 5, constitue un dispositif de régulation du niveau de pression dans la chambre 4 de combustion et se présente sous la forme d'un bouchon cylindrique enfoncé dans le canal du conduit formé par la cartouche 6. Ce bouchon comporte une paroi 610, perpendiculaire à l'axe du conduit, obturant le conduit, sur laquelle est formée une amorce 611 de rupture. L'amorce 611 de rupture constitue une zone de fragilisation suivant laquelle, sous une certaine pression des gaz, l'opercule 61 cède et s'ouvre en formant des pétales. Après l'ouverture de l'opercule, les pétales restent solidaires de l'opercule 61 ce qui permet d'éviter leur projection dans le reste du dispositif 1. Le seuil de claquage ou d'ouverture de l'opercule 61 frangible est déterminé par la profondeur de l'amorce 611 de rupture formée sur la paroi 610. La charge 62 pyrotechnique est placée dans le conduit formé par la cartouche 6 entre l'amorce 60 et l'opercule 61 frangible. La charge 62 pyrotechnique, avant que l'opercule 61 cède, est donc entièrement isolée du reste de la chambre 4 de combustion. L'opercule 61 cèdera par exemple une fois que la quasi-totalité de la charge 62 pyrotechnique aura brûlé. En réglant ainsi le seuil de claquage de l'opercule 61, la charge 62 pyrotechnique est maintenue dans un volume fermé et réduit au cours de sa combustion ce qui évite que certains grains de poudre soient projetés dans le reste de la chambre 4 de combustion et restent de ce fait imbrûlés. Ainsi cela permettra d'obtenir un rendement optimal et de conférer au dispositif 1 une grande fiabilité. Le deuxième sous-volume V11 du premier volume V1 de la chambre de combustion, défini dans le premier volume V1 par l'espace non-occupé par la cartouche 6 et situé en aval du premier sous-volume V10 c'est-à-dire en sortie de l'opercule 61 calibré, communique avec le deuxième volume V2 de la chambre de combustion par l'intermédiaire de l'ajutage 41. L'ajutage 41 constitue ainsi un deuxième dispositif de régulation du niveau de la pression des gaz dans la chambre 4 de combustion et permet de réduire la vitesse des gaz sortant de la cartouche 6. Selon l'invention, le ralentissement des gaz permet notamment de réduire le choc mécanique engendré, lors de l'injection, par le bouchon-piston aval 52 contre le système d'injection.

La forme en U du dispositif 1 et plus particulièrement le désalignement de la chambre 4 de combustion et du réservoir 5 de principe actif liquide permet également de casser l'onde de choc générée lors du début de la combustion de la charge 62 pyrotechnique.

Selon l'invention, la chambre 4 de combustion comporte un troisième volume V3 situé en aval du deuxième volume V2. Ce volume V3 est créé, lors du fonctionnement du dispositif 1, dans le réservoir 5 par les gaz de combustion. Ce volume V3 est formé plus précisément dans le tube 50 de verre entre le deuxième volume V2 et le bouchon-piston amont 51. Ce troisième volume V3 augmente donc en fonction du mouvement du bouchon-piston amont 51, ce mouvement étant créé lors du fonctionnement du dispositif 1 par les gaz de combustion. Ce troisième volume V3 est plus particulièrement délimité par une membrane 8, initialement repliée comme représenté en figure 6A, se déployant à l'intérieur du réservoir 5 sous l'action des gaz et poussant sous l'effet desdits gaz le bouchon-piston amont 51. Cette membrane 8 a la forme d'un capuchon fabriqué dans un matériau extensible, résistant à la chaleur et au vieillissement comme par exemple le caoutchouc. Selon l'invention, la présence de cette membrane 8 n'est pas obligatoire mais elle permet de limiter les risques de pollution du principe actif contenu dans le réservoir 5 par les gaz de combustion. Elle constitue donc une paroi étanche entre les gaz de combustion et le principe actif liquide. Selon l'invention, l'ajutage 41 est réalisé à travers la paroi 40 de manière à être le plus éloigné possible de la membrane 8 de sorte que les gaz chauds qui le traversent ne viennent pas trop en contact avec la membrane et ainsi ne l'endommagent pas. Comme représenté sur les figures 6A, 6B et 7, l'ajutage 41 est formé suivant un axe situé, lorsque le dispositif est posé sur son bouchon, dans un plan horizontal parallèle supérieur à celui comportant l'axe de la chambre 4 de combustion.

Le fonctionnement d'un tel dispositif 1 d'injection sans aiguille ayant des composants tels que ceux définis dans la présente demande est décrit en détail dans la demande de brevet français FR 2 815 544. Le fonctionnement global d'un tel dispositif 1 peut toutefois être résumé de la manière suivante :
Au repos, le percuteur 30 est par exemple en appui contre une butée à l'aide du ressort 31 précontraint dont l'axe est sensiblement confondu avec l'axe du percuteur 30. Une manipulation du patient provoque la libération du percuteur 30 qui, sous l'effet de la détente du ressort 31, vient percuter l'amorce 60 située dans le même axe. L'initiation de l'amorce 60 entraîne ensuite l'allumage de la charge 62 pyrotechnique contenue dans la cartouche 6. Lorsqu'une certaine pression de gaz est atteinte dans la cartouche 6 et lorsque le seuil de claquage de l'opercule 61 est atteint, l'opercule 61 frangible s'ouvre suivant son amorce 611 de rupture et laisse ainsi passer les gaz dans le deuxième sous-volume V11 du premier volume V1 de la chambre 4 de combustion. Les gaz présents dans le second sous-volume V11 sont ensuite ralentis lors de leur passage à travers l'ajutage 41 formé sur la paroi 40 séparant le premier volume V1 du deuxième volume V2 de la chambre 4 de combustion. Le réglage du diamètre de l'ajutage permet de maîtriser la mise en pression du deuxième volume V2 et ainsi par la suite de limiter les sollicitations sur la membrane 8. Les gaz présents dans le deuxième volume V2 pénètrent dans le troisième volume V3 formé perpendiculairement. Sous l'action des gaz, la membrane 8 se déplie et pousse, en se gonflant, sur le bouchon-piston amont 51 présent dans le tube 50 du réservoir 5. Le bouchon-piston amont 51 pousse à son tour le principe actif en direction du système d'injection et le principe actif est ainsi éjecté hors du dispositif 1.

Sur la figure 6B, le corps 2 représenté est celui d'un dispositif ayant déjà fonctionné, c'est-à-dire dans lequel la membrane 8 a été déployée et la cartouche 6, après la combustion de la totalité de la charge 62 pyrotechnique, a été vidée.

Selon l'invention, le premier sous-volume V10 aura par exemple un volume de 99 mm³, le second sous-volume V11 un volume de 149 mm³, le deuxième volume V2 un volume de 153 mm3 et le troisième volume V3 de 599 mm³. Dans une telle configuration, l'ajutage aura par exemple un diamètre de 2 mm.

Selon l'invention, la maîtrise successive du niveau de pression dans chacun des volumes V1,V2,V3 jusqu'au déploiement de la membrane 8 permet de contrôler les contraintes mécaniques, dynamiques et thermiques sur les composants qui délimitent la chambre 4 de combustion. Selon l'invention, les objectifs de coût, de masse et d'ergonomie du dispositif pourront donc être respectés. De plus la maîtrise successive du niveau de pression dans les différents volumes V1,V2,V3 permet également de contrôler le comportement mécanique et dynamique des éléments situés en aval de la chambre 4 de combustion dont dépend la performance de pénétration dans la peau.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif (1) d'injection sans aiguille jetable comprenant un corps (2) supportant et/ou délimitant une pluralité d'éléments formant un circuit d'éléments, ce circuit comprenant, de l'amont vers l'aval, un dispositif d'initiation associé à un générateur pyrotechnique de gaz, un réservoir (5) constitué d'un tube de verre (50) dans lequel sont enfoncés un bouchon-piston amont (51) et un bouchon-piston aval (52) emprisonnant un principe actif liquide à injecter et un système d'injection du principe actif, le générateur pyrotechnique de gaz comportant une charge (62) pyrotechnique placée dans une chambre (4) de combustion, ladite chambre (4) de combustion étant séparée en deux volumes (V1, V2) par une paroi (40) munie d'un passage (41), ces deux volumes (V1, V2) étant définis de l'amont vers l'aval comme un premier volume (V1) dans lequel est placé la charge pyrotechnique et un deuxième volume (V2), les deux volumes (V1, V2) communiquant par l'intermédiaire d'un premier dispositif de régulation du niveau de la pression dans la chambre (4) de combustion, ledit dispositif étant **caractérisé en ce que** ladite chambre comporte un troisième volume (V3) augmentant en fonction du mouvement dudit bouchon-piston amont (51), situé en aval du deuxième volume (V2), formé dans ledit tube (50) et délimité par une membrane (8) expansible mobile sous l'action des gaz issus de la combustion de la charge (62) pyrotechnique entre une position initialement repliée et une position déployée à l'intérieur dudit réservoir (5), ladite membrane constituant une paroi étanche entre ladite chambre de combustion (4)et ledit réservoir de principe actif (5).

2. Dispositif (1) selon la revendication 1, **caractérisé en ce que** la charge (62) pyrotechnique est disposée dans un premier sous-volume (V10) du premier volume (V1) de la chambre (4) de combustion, ce premier sous-volume (V10) étant initialement fermé.

3. Dispositif (1) selon la revendication 2, **caractérisé en ce que** le premier sous-volume (V10) du premier volume (V1) de la chambre (4) de combustion est séparé, par un deuxième dispositif de régulation du niveau de la pression, d'un deuxième sous-volume (V11) du premier volume (V1) de la chambre (4) de combustion, situé en aval par rapport au premier sous-volume (V10).

4. Dispositif (1) selon la revendication 3, **caractérisé en ce que** le deuxième dispositif de régulation est constitué d'un opercule (61) frangible calibré.

5. Dispositif (1) selon la revendication 4, caractérisé ce que le premier sous-volume (V10) du premier volume (V1), dans lequel est placée la charge (62) pyrotechnique, est délimité en partie par les parois d'une cartouche (6) insérée dans le corps (2) du dispositif (1).

6. Dispositif (1) selon la revendication 5, **caractérisé en ce que** la charge (62) pyrotechnique est placée dans la cartouche (6) entre l'opercule (61) frangible calibré et une amorce (60) apte à initier la charge (62) pyrotechnique.

7. Dispositif (1) selon la revendication 6, **Caractérisé en ce que** la cartouche (6) a la forme d'un conduit en L dans lequel est placée la charge (62) pyrotechnique, ce conduit étant obturé à l'une de ses extrémités par l'amorce (60) et à son autre extrémité par l'opercule (61) frangible calibré.

8. Dispositif (1) selon l'une quelconque des revendications précédentes, **caractérisé en ce que** ledit passage (41) est décalé par rapport à un axe central longitudinal de la chambre (4) de combustion et est formé de sorte à être le plus éloigné possible de la membrane (8).

## Patentansprüche

1. Wegwerfbare Injektionsvorrichtung (1) ohne Nadel, die einen Körper (2) umfasst, der eine Mehrzahl von Elementen stützt und/oder abgrenzt, die einen Kreislauf von Elementen bilden, wobei dieser Kreislauf von stromaufwärts nach stromabwärts eine Initiierungsvorrichtung, die mit einem pyrotechnischen Gasgenerator assoziiert ist, einen Behälter (5), der aus einer Glasröhre (50) besteht, in die ein stromaufwärtiger Stopfen-Kolben (51) und ein stromabwärtiger Stopfen-Kolben (52) gedrückt sind, die einen zu injizierenden flüssigen Wirkstoff und ein Injektionssystem des Wirkstoffs einschließen, umfasst, wobei der pyrotechnische Gasgenerator eine pyrotechnische Ladung (62) umfasst, die in einer Brennkammer (4) platziert ist, wobei die Brennkammer (4) in zwei Volumen (V1, V2) durch eine Wand (40) getrennt ist, die mit einem Durchgang (41) versehen ist, wobei diese zwei Volumen (V1, V2) von stromaufwärts nach stromabwärts als ein erstes Volumen (V1), in dem die pyrotechnische Ladung platziert ist, und ein zweites Volumen (V2) definiert sind, wobei die zwei Volumen (V1, V2) über eine erste Regelvorrichtung des Druckpegels in der Brennkammer (4) kommunizieren, wobei die Vorrichtung **dadurch gekennzeichnet ist, dass** die Kammer ein drittes Volumen (V3) umfasst, das in Abhängigkeit von der Bewegung des stromabwärtigen Stopfen-Kolbens (51), der stromabwärts des zweiten Volumens (V2) liegt, zunimmt, das in der zweiten Röhre (50) gebildet ist und von einer dehnbaren Membran (8) abgegrenzt ist, die unter der Einwirkung der Gase, die aus der Verbrennung der pyrotechnischen Ladung (62) hervorgehen, zwischen einer anfänglich zurückgezogenen und einer ausgefahrenen Position in dem Inneren des Behälters (5) beweglich ist, wobei die Membran eine dichte Wand zwischen der Brennkammer (4) und dem Wirkstoffbehälter (5) bildet.

2. Vorrichtung (1) nach Anspruch 1, **dadurch gekennzeichnet, dass** die pyrotechnische Ladung (62) in einem ersten Subvolumen (V10) des ersten Volumens (V1) der Brennkammer (4) angeordnet ist, wobei dieses erste Subvolumen (V10) anfänglich geschlossen ist.

3. Vorrichtung (1) nach Anspruch 2, **dadurch gekennzeichnet, dass** das erste Subvolumen (V10) des ersten Volumens (V1) der Brennkammer (4) von einer zweiten Regelvorrichtung des Pegels des Drucks von einem zweiten Subvolumen (V11) des ersten Volumens (V1) der Brennkammer (4), das in Bezug auf das erste Subvolumen (V10) stromabwärts liegt, getrennt ist.

4. Vorrichtung (1) nach Anspruch 3, **dadurch gekennzeichnet, dass** die zweite Regelvorrichtung aus einem kalibrierten splitterbaren Verschlusselement (61) besteht.

5. Vorrichtung (1) nach Anspruch 4, **dadurch gekennzeichnet, dass** das erste Subvolumen (V10) des ersten Volumens (V1), in dem die pyrotechnische Ladung (62) platziert ist, teilweise von den Wänden einer Patrone (6), die in den Körper (2) der Vorrichtung (1) eingesetzt ist, abgegrenzt ist.

6. Vorrichtung (1) nach Anspruch 5, **dadurch gekennzeichnet, dass** die pyrotechnische Ladung (62) in der Patrone (6) zwischen dem kalibrierten splitterbaren Verschlusselement (61) und einem Zünder (60), der geeignet ist, die pyrotechnische Ladung (62) zu zünden, platziert ist.

7. Vorrichtung (1) nach Anspruch 6, **dadurch gekennzeichnet, dass** die Patrone (6) die Form einer L-Leitung hat, in der die pyrotechnische Ladung (62) platziert ist, wobei diese Leitung an einem ihrer Enden durch den Zünder (60) und an ihrem anderen Ende durch das kalibrierte splitterbare Verschlusselement (61) verschlossen ist.

8. Vorrichtung (1) nach einem der vorhergehenden Ansprüche, **dadurch gekennzeichnet, dass** der Durchgang (41) in Bezug auf eine zentrale Längsachse der Brennkammer (4) versetzt und derart gebildet ist, dass sie möglichst weit von der Membran (8) entfernt ist.

## Claims

1. A disposable needleless injection device (1) comprising a body (2) supporting and/or delimiting a plurality of elements forming a circuit of elements, this circuit comprising, from upstream to downstream, an initiating device associated to a pyrotechnic gas generator, a reservoir (5) constituted by a glass tube (50) in which are pushed in an upstream plunger stopper (51) and a downstream plunger stopper (52) confining a liquid active ingredient to be injected and a system for injecting the active ingredient, the pyrotechnic gas generator including a pyrotechnic charge (62) placed in a combustion chamber (4), said combustion chamber (4) being separated into two volumes (V1, V2) by a wall (40) provided with a passage (41), these two volumes (V1, V2) being defined from upstream to downstream as a first volume (V1) in which is placed the pyrotechnic charge and a second volume (V2), the two volumes (V1, V2) communicating via a first device for regulating the level of the pressure in the combustion chamber (4), said device being **characterized in that** said chamber includes a third volume (V3) increasing according to the movement of said upstream plunger stopper (51), located downstream of the second volume (V2), formed in said tube (50) and delimited by an expandable diaphragm (8) movable by the action of the gases originating from the combustion of the pyrotechnic charge (62) between an initially retracted position and a position deployed inside said reservoir (5), said diaphragm constituting a tight wall between said combustion chamber (4) and said active ingredient reservoir (5).

2. The device (1) according to claim 1, **characterized in that** the pyrotechnic charge (62) is disposed in a first sub-volume (V10) of the first volume (V1) of the combustion chamber (4), this first sub-volume (V10) being initially closed.

3. The device (1) according to claim 2, **characterized in that** the first sub-volume (V10) of the first volume (V1) of the combustion chamber (4) is separated, by a second device for regulating the level of the pressure, from a second sub-volume (V11) of the first volume (V1) of the combustion chamber (4), located downstream with respect to the first sub-volume (V10).

4. The device (1) according to claim 3, **characterized in that** the second regulation device is constituted by a calibrated frangible cap (61).

5. The device (1) according to claim 4, **characterized in that** the first sub-volume (V10) of the first volume (V1), in which the pyrotechnic charge (62) is placed, is partially delimited by the walls of a cartridge (6) inserted into the body (2) of the device (1).

6. The device (1) according to claim 5, **characterized in that** the pyrotechnic charge (62) is placed in the cartridge (6) between the calibrated frangible cap (61) and a primer (60) capable of initiating the pyrotechnic charge (62).

7. The device (1) according to claim 6, **characterized in that** the cartridge (6) is in the form of an L-shaped conduit in which the pyrotechnic charge (62) is placed, this conduit being sealed at one of its ends by the primer (60) and at its other end by the calibrated frangible cap (61).

8. The device (1) according to any one of the preceding claims, **characterized in that** said passage (41) is shifted with respect to a longitudinal central axis of the combustion chamber (4) and is formed so as to be as far away as possible from the diaphragm (8).
